# EUROPEAN PATENT SPECIFICATION

(11) **EP 1 148 377 B1**
(45) Date of publication and mention of the grant of the patent: **10.11.2004**
(21) Application number: 01200903.1
(22) Date of filing: 12.03.2001
(51) Int. Cl.: G03B 42/04, G03C 3/00

(54) **Intraoral dental radiographic film packet with formed comfort enhancing perimeter**
Intraorale Dentalröntgenfilmpackung mit geformtem Rand zur Verbesserung des Komforts
Paquet pour un film radiographique dentaire intra-oral avec un périmètre fabriqué pour améliorer le confort

(30) Priority: 24.03.2000 US 534372; 24.03.2000 US 533867; 24.03.2000 US 533868; 24.03.2000 US 534368; 24.03.2000 US 534370; 24.03.2000 US 534392; 24.03.2000 US 534393; 24.03.2000 US 534516
(43) Date of publication of application: 24.10.2001
(73) Proprietor: EASTMAN KODAK COMPANY, Rochester, New York 14650 (US)
(72) Inventor: Bacchetta, Richard W., Rochester, New York 14650-2201 (US); Mcgovern, Michael R., Rochester, New York 14650-2201 (US); Schwallie, Scott H., Rochester, New York 14650-2201 (US); He, Fugui, Rochester, New York 14650-2201 (US); Resch, Douglas T., Rochester, New York 14650-2201 (US); Richter, Edward B., Rochester, New York 14650-2201 (US); Konte, Bruce W., Rochester, New York 14650-2201 (US); Merz, Gary E., Rochester, New York 14650-2201 (US); Earnhart, Edgar G., Rochester, New York 14650-2201 (US); Allen, David C., Rochester, New York 14650-2201 (US)
(74) Representative: Haile, Helen Cynthia

(56) References cited:
- US-A- 4 912 740

## Description

The present invention relates generally to x-ray film packets and, in particularly, to intraoral radiographic film packets with comfort enhancing features.

A common problem experienced by people visiting the dentist is the discomfort and pain associated with the taking of dental x-rays caused by the positioning of intraoral radiographic film packets in the patient's mouth. The typical intraoral rear graphic film packet includes relatively hard and/or sharp edges which press against and irritate the gums and other oral soft tissue of the person whose teeth are being x-rayed. A variety of intraoral x-ray dental packets are known in the prior art which include features intended to be comfort enhancing. In addition, attempts have been made to create comfort enhancing structures into which intra-x-ray dental packets can be inserted prior to placement in the patient's mouth. One example of this type of structure is taught in U.S. Patent No. 5,044,008 titled "Dental Film Cartridge Cushion," by Reginald B. Jackson, August 27, 1991. Jackson utilizes a cartridge cushion comprising a foam sheet sandwich into which the x-ray dental packet is placed for the purpose of cushioning and increasing the comfort to the patient. Jackson requires the manual insertion of the x-ray packet into the cartridge cushion. Thus, Jackson adds significant bulk to the packet and enhances the possibility of triggering a gag reflex action in the patient. Additionally, after the cartridge cushion is removed from the packet, it would be possible to reuse the cartridge cushion which would not be sanitary.

A second example of an add-on structure is taught in U.S. Patent No. 5,285,491 titled "Dental Film Packet," by Wilfried Muylle et al., February 8, 1994. Muylle et al. teaches sealing a film pack in an envelope consisting of a pair of thin pockets of injection molded plastic which are sealed with a band of adhesive tape. The envelope has no sharp edges and generally rounded corners. Thus, as with Jackson's device, this device requires manual insertion of the packet, adds significant bulk to the packet, enhances the possibility of triggering a gag reflex in the patient, and can also be reused in a non-sanitary manner.

U.S. Patent No. 1,631,497 titled "Dental X-ray Film Package," by Harry L. Marler, June 7, 1927, teaches a dental x-ray film package wherein a sensitized sheet is sandwiched between two opaque sheets. A heavy band of rubber is stretched about the periphery of the package to hold the package securely together and to provide the light-tight joint.

U.S. Patent. No. 1,537,925 titled "Dental X-ray Film Package," by Leonard M. Bolin, May 12, 1925, teaches a dental x-ray film package wherein a pair of film sheets and the cover sheet are inserted into the container. The container consists of a frame including a backing portion in an enlarged continuous beading about the periphery thereof. The beading must be forced away from the backing portion and stretched peripherally in order to insert the film sheets and cover sheet therein. The container thus serves to hold the package together and provide the light seal.

U.S. Patent No. 4,791,657 titled "Intraoral Radiographic Film Packet," by Alan Kirsch et al., December 13, 1988, teaches a dental radiographic film packet which includes soft comers for greater patient comfort. The packet is constructed by removing all material from the comers of a typical dental radiographic film packet with the exception of the film chip. Individual comer covers which are seamless pockets are then added to the four comers of the packet. The comer covers create an airspace at each comer around the edge of the film chip.

U.S. Patent No. 2,084,092 titled "Intraoral Radiographic Film Packet," by Alan Kirsch et al., December 13, 1988, teaches a dental film holder that is a stretchable vellum rubber plate with integral comer pockets into which an x-ray dental packet may be manually inserted. Kenney's dental film holder is intended to be reusable.

Another conventional intraoral x-ray film packet is disclosed in US-A-4 912 740.

From the foregoing it can be seen that many attempts to add a comfort enhancing feature to dental x-ray film packets have resulted in structures requiring manual assembly and/or modification of individual film packets in order to receive comfort enhancing structure. Further, such prior art attempts have failed to provide a dental x-ray film packet in which the comfort enhancing feature is formed integrally therewith during manufacture and without requiring manual assembly.

It is therefore an object of the present invention to provide an intraoral radiographic film packet with a comfort enhancing perimeter as defined in claim 1.

The intraoral radiographic film packet has a comfort enhancing perimeter integrally formed therewith.

The intraoral radiographic film packet includes a comfort enhancing perimeter which does not significantly increase the bulk of the film packet.

The comfort enhancing perimeter feature for an intraoral radiographic film packet ensure that it cannot be reused in a non-sanitary manner.

The foregoing and numerous other features, objects and advantages of the present invention will become readily apparent upon a review of the detailed description, claims and drawings set forth herein. These features, objects and advantages are accomplished by forming each of the two outer thermoplastic layers of the x-ray packet with a perimetric hollow semicylindrical feature. Sandwiched between these two outer thermoplastic layers are the typical elements comprising a dental film pack including a paper wrap, the film chip and a lead shield. With these typical elements sandwiched between the two outer thermoplastic layers, the two outer thermoplastic layers are sealed to one another both inside and outside the perimetric cylinder formed by the interface of the two perimetric semicylindrical features. Sealing can be accomplished by heat sealing, RF sealing, or any other type of sealing which would yield a substantially airtight perimetric cylindrical chamber. In such manner, the dental film packet is created which includes an integrally formed perimetric cushion.
Figure 1 is a cross-sectional view of a typical prior art dental film packet.
Figure 2 is a perspective view of the intraoral radiographic film packet with comfort enhancing features of the present invention.
Figure 3 is a partially exploded cross-sectional view of the intraoral radiographic film packet of the present invention taken along line 3-3 of Figure 2.
Figure 4 is a partial cross-sectional view of the intraoral radiographic film packet of the present invention taken along line 4-4 of Figure 2.

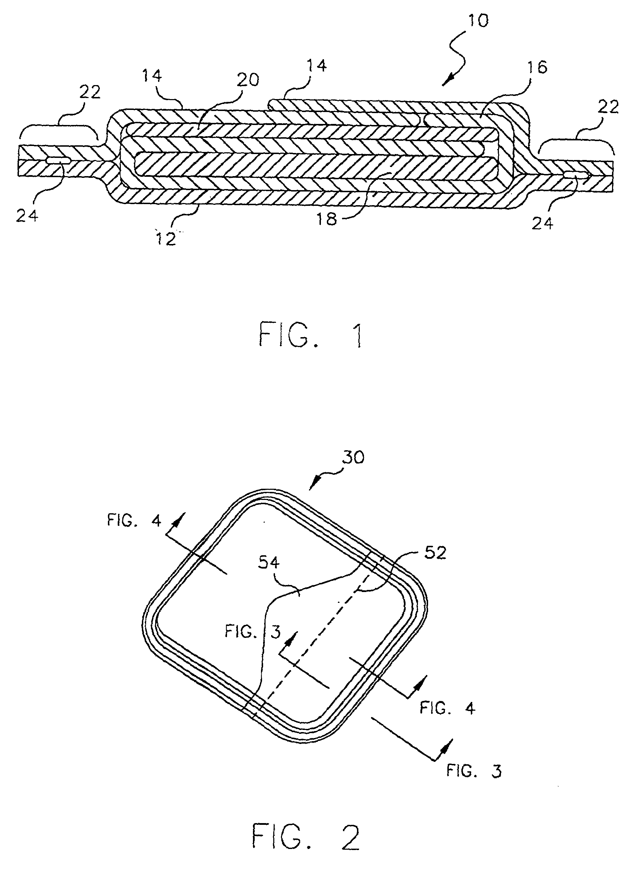

Turning first to Figure 1, there is shown a cross-section of a typical prior art dental film packet 10. Dental film packet 10 includes an outer envelope comprising a vinyl sheet 12 on one face of the dental film packet 10 and a pair of overlapping vinyl sheets 14 on the opposite face thereof. Contained between the sheet 12 and overlapping sheets 14 are a paper wrap element 16, a film chip 18 and a lead foil 20. Vinyl sheets 12 and 14 project beyond dimensions of the paper wrap element 16, the film chip 18 and lead foil 20 to yield a perimetric edge 22. Laminated perimetric edge 22 allows for heat sealing of vinyl sheets 12 and 14 to one another to yield a light tight perimeter to the dental film packet 10. In addition, a heat seal 24 is generated at the overlap of vinyl sheets 14 to provide an outer envelope which is completely light-tight and which is substantially watertight. This prior art dental film packet 10 therefore includes a laminated, relatively stiff and sharp perimetric edge created by the laminated perimetric edge 22. It is this relatively stiff and sharp perimetric edge which causes discomfort to the patient.

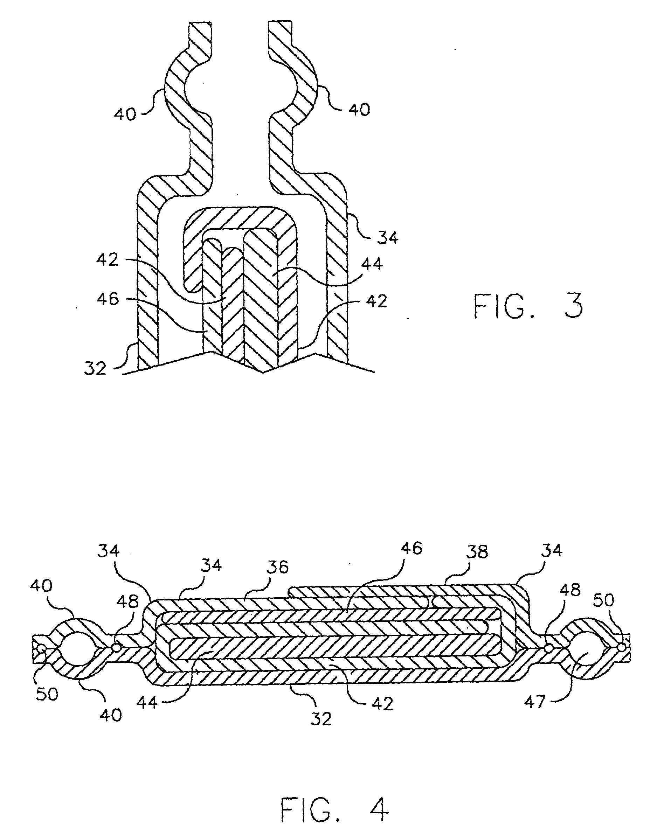

Turning next to Figures 2, 3 and 4, there is shown the dental film packet 30 of the present invention. Dental film packet 30 includes an envelope comprising a first outer sheet 32 and an opposing second outer sheet 34. Second outer sheet 34 is actually comprised of a pair of overlapping sections 36 and 38. Each outer sheet 32 and 34 includes a perimetric generally semicylindrical element 40 formed therein. The forming of outer sheets 32 and 34 can be by a variety of techniques including vacuum forming, molding, embossing, or the like. Outer sheets 32 and 34 are preferably made of a soft thermoplastic material such as, but not limited to polyvinyl chloride (PVC), or ethylene vinyl acetate (EVA). Sandwiched between outer sheets 32 and34 are the typical elements found in a dental x-ray film packet. There is a paper wrap element 42, the film chip 44 and a lead foil 46. The dental x-ray film packet 30 is assembled by placing the paper wrap element 42, the film chip 44 and a lead foil 46 between outer sheets 32 and 34 and sealing the outer sheets 32 and 34 to one another about the perimeter. There are actually two perimetric seals generated (an inner seal and an outer seal, indicated by spots 48 and 50, respectively), one on each side of cylindrical air cushion 47 formed by the interface of the two semicylindrical elements 40. This air cushion 47 provides a comfort edge for the patient. Inner seal 48 and outer seal 50 can be formed by heat sealing, RF sealing, or any other sealing mechanism which can create a substantially airtight bond between outer sheets 32 and 34. There is also a transverse seal (indicated by line 52) affixing overlapping sections 36 and 38 together. Overlapping section 38 is preferably formed with a tab portion 54 which extends past transverse seal 52 to facilitate removal of the envelope for extraction and development of the film chip 44 after exposure.

Those skilled in the art will also recognize that there are now digital radiography products available which are intended to be used in place of dental x-ray film packets. One example of this type of technology uses a plate that is coated with phosphorous. When exposed to radiation, the plate will create an image that can be scanned with a laser into a computer instead of being chemically processed. To the extent that these products have the same problems of patient discomfort, the present invention can be used to solve such problems. Similarly, intraoral products which use a CCD sensor array may also achieve some level of comfort benefit through the application of the present invention.

## Claims

1. An intraoral x-ray film packet (30) comprising:
(a) a first outer sheet (32) having a first generally semicylindrical perimetric element (40) formed therein;
(b) a second outer sheet (34) including a second generally semicylindrical perimetric element (40) formed therein corresponding to the first generally semicylindrical perimetric element, said first and second generally semicylindrical perimetric elements are aligned to form a perimetric cushion (47) when the first and second outer sheets are affixed together;
(c) a film chip (44) residing between said first outer sheet and said second outer sheet;
(d) a first continuous perimetric seal (50) affixing said first outer sheet and said second outer sheet and positioned externally of said perimetric cushion; and
(e) a second continuous perimetric seal (48) affixing said first outer sheet and said second outer sheet and positioned between said perimetric cushion and said film chip.

2. An intraoral x-ray film packet as recited in claim 1 wherein: said second outer sheet comprises a pair of overlapping sections (36,38).

3. An intraoral x-ray film packet as recited in claim 2 wherein: said second outer sheet further comprises a transverse seal (52) affixing said pair of overlapping sections.

4. An intraoral x-ray film packet as recited in claim 3 wherein: one of said pair of said pair of overlapping sections includes a tab portion (54) to facilitate removal of said first and second outer sheets.

5. An intraoral x-ray film packet as recited in claim 1 wherein: said first and second outer sheets are made from a thermoplastic material.

6. An intraoral x-ray film packet as recited in claim 1 wherein: said first and second perimetric seal are formed by RF sealing.

7. An intraoral x-ray film packet as recited in claim 1 wherein: said first and second generally semicylindrical perimetric elements are formed by vacuum forming.

8. An intraoral x-ray film packet as recited in claim 1 wherein: said first and second generally semicylindrical perimetric elements are formed by molding.

9. An intraoral x-ray film packet as recited in claim 1 wherein: said first and second generally semicylindrical perimetric elements are formed by embossing.

## Patentansprüche

1. Intraoraler Röntgenfilmpack (30) mit
a) einem ersten äußeren Mantel (30), der ein erstes, im wesentlichen halbzylindrisch ausgebildetes, perimetrisches Element (40) aufweist;
b) einem zweiten äußeren Mantel (34), der ein zweites, im wesentlichen halbzylindrisch ausgebildetes, perimetrisches Element (40) aufweist, welches dem ersten, im wesentlichen halbzylindrisch ausgebildeten Element entspricht, wobei die ersten und zweiten, im wesentlichen halbzylindrisch ausgebildeten Elemente ausgerichtet sind, um ein perimetrisches Kissen (47) zu bilden, wenn der erste und zweite äußere Mantel verbunden sind;
c) einem zwischen dem ersten äußeren Mantel und dem zweiten äußeren Mantel angeordneten Filmchip;
d) einem ersten durchgehenden, perimetrischen Versiegelungsmittel (50), das den ersten äußeren Mantel und den zweiten äußeren Mantel verbindet und außerhalb des perimetrischen Kissens angeordnet ist; und
e) einem zweiten durchgehenden, perimetrischen Versiegelungsmittel (48), das den ersten äußeren Mantel und den zweiten äußeren Mantel verbindet und zwischen dem perimetrischen Kissen und dem Filmchip angeordnet ist.

2. Intraoraler Röntgenfilmpack nach Anspruch 1, **dadurch gekennzeichnet, dass** der zweite äußere Mantel (34) zwei sich überlappende Abschnitte (36, 38) aufweist.

3. Intraoraler Röntgenfilmpack nach Anspruch 2, **dadurch gekennzeichnet, dass** der zweite äußere Mantel (34) zusätzlich ein quer verlaufendes Versiegelungsmittel (52) aufweist, welches die beiden sich überlappenden Abschnitte verbindet.

4. Intraoraler Röntgenfilmpack nach Anspruch 3, **dadurch gekennzeichnet, dass** einer der beiden sich überlappenden Abschnitte eine Lasche (54) aufweist, die das Entfernen des ersten und zweiten äußeren Mantels vereinfacht.

5. Intraoraler Röntgenfilmpack nach Anspruch 1, **dadurch gekennzeichnet, dass** der erste und zweite äußere Mantel aus thermoplastischem Material besteht.

6. Intraoraler Röntgenfilmpack nach Anspruch 1, **dadurch gekennzeichnet, dass** das erste und zweite perimetrische Versiegelungsmittel durch HF-Versiegelung gebildet wird.

7. Intraoraler Röntgenfilmpack nach Anspruch 1, **dadurch gekennzeichnet, dass** das erste und zweite, im wesentlichen halbzylindrisch ausgebildete, perimetrische Element durch Unterdruckverformung gebildet wird.

8. Intraoraler Röntgenfilmpack nach Anspruch 1, **dadurch gekennzeichnet, dass** das erste und zweite, im wesentlichen halbzylindrisch ausgebildete, perimetrische Element durch Formgießen gebildet wird.

9. Intraoraler Röntgenfilmpack nach Anspruch 1, **dadurch gekennzeichnet, dass** das erste und zweite, im wesentlichen halbzylindrisch ausgebildete, perimetrische Element durch Pressen gebildet wird.

## Revendications

1. Conditionnement de film radiographique intraoral (30) comprenant :
(a) une première feuille extérieure (32) comportant un premier élément périmétrique généralement demi-cylindrique (40) formé dans celle-ci,
(b) une seconde feuille extérieure (34) comprenant un second élément périmétrique généralement demi-cylindrique (40) formé dans celle-ci correspondant au premier élément périmétrique généralement demi-cylindrique, lesdits premier et second éléments périmétriques généralement demi-cylindriques sont alignés pour former un coussin périmétrique (47) lorsque les première et seconde feuilles extérieures sont fixées ensemble,
(c) un morceau de film (44) se trouvant entre ladite première feuille extérieure et ladite seconde feuille extérieure,
(d) un premier joint périmétrique continu (50) attachant ladite première feuille extérieure et ladite seconde feuille extérieure et positionné à l'extérieur dudit coussin périmétrique, et
(e) un second joint périmétrique continu (48) attachant ladite première feuille extérieure et ladite seconde feuille extérieure et positionné entre ledit coussin périmétrique et ledit morceau de film.

2. Conditionnement de film radiographique intraoral selon la revendication 1, dans lequel :
ladite seconde feuille extérieure comprend une paire de sections en chevauchement (36, 38).

3. Conditionnement de film radiographique intraoral selon la revendication 2, dans lequel :
ladite seconde feuille extérieure comprend en outre un joint transversal (52) attachant ladite paire de sections en chevauchement.

4. Conditionnement de film radiographique intraoral selon la revendication 3, dans lequel :
l'une de ladite paire des sections en chevauchement comprend une partie de languette (54) pour faciliter l'enlèvement desdites première et seconde feuilles extérieures.

5. Conditionnement de film radiographique intraoral selon la revendication 1, dans lequel :
lesdites première et seconde feuilles extérieures sont constituées d'un matériau thermoplastique.

6. Conditionnement de film radiographique intraoral selon la revendication 1, dans lequel:
lesdits premier et second joints périmétriques sont formés par scellement haute fréquence.

7. Conditionnement de film radiographique intraoral selon la revendication 1, dans lequel :
lesdits premier et second éléments périmétriques généralement demi-cylindriques sont formés par formage sous vide.

8. Conditionnement de film radiographique intraoral selon la revendication 1, dans lequel :
lesdits premier et second éléments périmétriques généralement demi-cylindriques sont formés par moulage.

9. Conditionnement de film radiographique intraoral selon la revendication 1, dans lequel :
lesdits premier et second éléments périmétriques généralement demi-cylindriques sont formés par emboutissage.
